# FASCICULE DE BREVET EUROPEEN

(11) **EP 1 140 825 B1**
(45) Date de publication et mention de la délivrance du brevet: **07.05.2003**
(21) Numéro de dépôt: 99961092.6
(22) Date de dépôt: 16.12.1999
(51) Int. Cl.: C07D 209/16, A61K 31/40

(54) **DERIVES DE MELATONINE ET MEDICAMENT COMPRENANT DE TELS DERIVES**
MELATONINDERIVATE UND DIESE ENTHALTENDE PHARMAZEUTISCHE ZUBEREITUNG
MELATONIN DERIVATIVES AND MEDICINE CONTAINING SAME

(30) Priorité: 18.12.1998 FR 9816015
(43) Date de publication de la demande: 10.10.2001
(73) Titulaire: CENTRE NATIONAL DE LA RECHERCHE SCIENTIFIQUE (CNRS), 75794 Paris Cedex 16 (FR)
(72) Inventeur: DUCROCQ, Claire, F-91400 Orsay (FR); BLANCHARD, Béatrice, F-92500 Rueil-Malmaison (FR)
(74) Mandataire: Warcoin, Jacques
(86) Numéro de dépôt international: FR9903163
(87) Numéro de publication internationale: WO00037441

(56) Documents cités:
- EP-A- 0 513 702
- EP-A- 0 543 659
- WO-A-97/06140
- WO-A-98/09653
- FR-A- 2 753 095

## Description

La présente invention concerne de nouveaux dérivés de mélatonine susceptibles de libérer dans les conditions physiologiques le monoxyde d'azote (NO) et la mélatonine.

Elle a également pour objet ces dérivés de la mélatonine pour leur application en tant que substances thérapeutiquement actives.

Le monoxyde d'azote est un produit des NO-synthétases dans tous les organes animaux, où il joue des rôles variés dans le maintien des fonctions physiologiques mais surtout dans les situations pathologiques où il est synthétisé à des quantités bien supérieures. C'est un agent vasodilatateur, anti-aggrégant des plaquettes et des leucocytes avec d'autres types cellulaires. Il intervient dans les transmissions nerveuses, ni cholinergiques, ni adrénergiques, mais aussi dans les processus d'excitotoxicité du glutamate dans le système nerveux. Dans les processus neurodégénératifs, il est encore difficile de faire la part de l'intervention de NO et/ou des dérivés de l'oxygène. D'après plusieurs auteurs, NO aurait plutôt un rôle protecteur des neurones et aurait un rôle antioxydant en se combinant aux radicaux oxygénés. Cependant lorsqu'il se lie au superoxyde, il génère le peroxynitrite qui à son tour réalise des modifications de macromolécules et tout particulièrement de l'ADN. La fragmentation de l'ADN constitue une phase du processus d'apoptose.

Comme NO est un excellent piège des anions superoxyde en générant le peroxynitrite, la protection des tissus ne peut être assurée qu'en présence de composés susceptibles de piéger le peroxynitrite, tels que la mélatonine.

La mélatonine ou N-acétyl-5-méthoxytryptamine est une hormone synthétisée dans la glande pinéale et dans la rétine surtout pendant la nuit et chez les jeunes animaux. Son niveau décroît avec l'âge et au cours du développement de la maladie d'Alzheimer. Elle contrôle les fonctions physiologiques associées au rythme circadien et intervient dans la réponse immune.

Récemment, il a été proposé que son rôle essentiel soit de piéger les radicaux et les forts oxydants. Ces interactions n'exigent pas de reconnaissance avec des récepteurs spécifiques mais interviennent dans les processus physiopathologiques du monde vivant. Cette hypothèse est basée sur des résultats expérimentaux directs mais aussi sur les effets de protection par la mélatonine des dommages oxydatifs des protéines, lipides membranaires et ADN, dus à des processus radicalaires. En particulier, la mélatonine empêche généralement la peroxydation lipidique *in vitro* comme *in vivo.*

Le radical hydroxyle est l'espèce toxique majeure provenant de l'oxygène chez les organismes vivants. Son taux augmente avec l'âge. Il est responsable de l'initiation de réactions radicalaires en chaîne qui caractérisent le stress oxydatif. Il n'existe pas de système enzymatique spécifique capable de l'inactiver comme c'est le cas pour l'anion superoxyde. C'est le rôle des antioxydants (vitamines C et E, le glutathion, le carotène, etc.). La mélatonine en est parmi les plus efficaces puisqu'elle réagit avec ce radical avec une vitesse proche de la limite de diffusion. Par contre, elle ne réagit pas ou très lentement avec l'anion superoxyde.

Elle intervient aussi dans l'équilibre oxydoréductif en maintenant l'homéostasie du glutathion et en initiant la synthèse de gènes d'enzymes antioxydants.

Ses propriétés permettent d'expliquer pourquoi la mélatonine réduit la toxicité du glutamate dans les cultures neuronales et les effets indésirables au cours de chimiothérapies anticancéreuses. Dans ce cas, ces effets thérapeutiques s'ajoutent à ses propriétés oncostatiques observées sur des cultures de cellules humaines cancéreuses et sur certains cancers.

Enfin, des résultats récents semblent encourageants pour utiliser la mélatonine dans la prophylaxie de l'apparition des ulcères gastriques et de la migraine.

Parmi les donneurs de NO connus actuellement, seuls les diazénium diolates, appelés généralement NONOates libèrent spontanément et quantitativement leurs groupements nitrosyle en NO.

Les autres donneurs de NO libèrent NO après des étapes réductrices ou oxydantes. Les seuls utilisés comme médicaments vasodilatateurs dans les maladies cardio-vasculaires, les nitrates organiques et les sydnonymines présentent des mécanismes de dégradation complexes *in vitro* et mal connus *in vivo.* Les nitrates organiques sont métabolisés au niveau de la paroi des vaisseaux sanguins. La molsidomine ou pirsidomine sont hydrolysées par voie enzymatique dans le foie et libèrent des composés actifs engendrant spontanément NO ainsi que l'anion superoxyde, *in vitro.*

Toutefois, aucun de ces composés ne libère simultanément un antioxydant susceptible notamment de piéger le peroxynitrite et le monoxyde d'azote.

On connaît par ailleurs par les demandes de brevet EP-A-513 702 et EP-A-543 659 des dérivés de mélatonine utilisés comme médicaments.

Il est donc souhaitable de réaliser des composés capables à la fois de relarguer les deux antioxydants : le monoxyde d'azote et un antioxydant notamment susceptible de piéger le peroxynitrite. De plus, de tels composés doivent présenter un caractère lipophile suffisant pour être physiologiquement acceptable notamment pour franchir la barrière hématoencéphalique.

Les composés objet de la présente invention résolvent simultanément les problèmes mentionnés ci-dessus.

Selon l'invention, le dérivé de mélatonine répond à la formule : 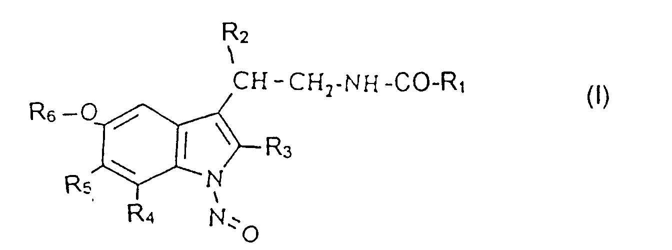 dans laquelle :
R₁ représente H, un groupe alkyle en C₁ à C₄ ou un groupe alcoxy en C₁ à C₄.
R₂ représente H ou un groupe alkyle en C₁ à C₄
R₃ représente H, méthyle ou un atome d'halogène,
R₄, R₅ représentent individuellement H ou un atome d'halogène,
R₆ représente H ou un groupe alkyle en C₁-C₄
ou un sel pharmaceutiquement acceptable de celui-ci.

De préférence R₂= H ; R₆ = CH₃ ; R₁ = CH₃.

Selon une variante préférée de l'invention, le dérivé de mélatonine répond à la formule :

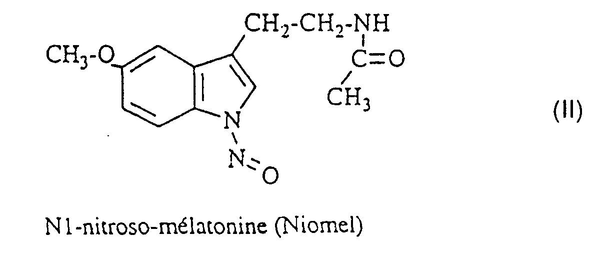

L'invention a également pour objet les composés de formule I ou II pour leur application en tant que substances thérapeutiquement actives.

Elle a également pour objet une composition pharmaceutique comprenant un composé de formule I ou II ainsi qu'un excipient pharmacologiquement acceptable.

Parmi les applications thérapeutiques, on cite les maladies cardio-vasculaires, les ischémie-reperfusions, les maladies parasitaires, les maladies inflammatoires, les maladies neurodégénératives notamment la maladie d'Alzheimer, les myopathies, les anémies falciformes.

L'invention a également pour objet un procédé de préparation des dérivés de mélatonine de formule I, caractérisé qu'on met en contact en solution organique protique un précurseur de formule : 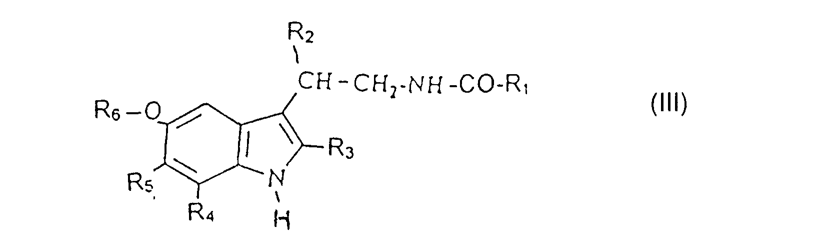 dans laquelle R₁ à R₆ ont les mêmes significations que celles indiquées pour la formule I,
avec une solution aqueuse de nitrite de sodium puis on alcalinise de façon à obtenir un dérivé selon l'invention.

Le dérivé de mélatonine de formule II est préparé de la façon suivante :

A une solution de mélatonine dans l'acide acétique et le méthanol, refroidie à 4°C, on ajoute une solution aqueuse de nitrite de sodium. Après alcalinisation par une solution saturée de bicarbonate de sodium, la N1-nitrosomélatonine est extraite au dichlorométhane, la solution organique est séchée par du sulfate de magnésium, puis évaporée sous pression réduite. Le résidu est repris dans l'acétate d'éthyle et l'hexane jusqu'à l'obtention de cristaux. Les cristaux sont récupérés par filtration, séchés et conservés à l'abri de la lumière à température ambiante.

Ce composé présente un point de fusion de : 138 °C.

Une analyse chromatographique sur colonne Hypersil C18, élution à l'aide d'un gradient de 10 à 50 % d'acétonitrile et 0,05 % d'acide trifluoroacétique en 50 minutes, détection 215 nm, conduit à un pic à 431, alors que le pic de la mélatonine est à 258.

La bande caractéristique du spectre d'absorption lumineuse du produit en solution phosphate de pH 4 à pH 9 est située à 346 nm.

On observe un pic remarquable (m/z M + H⁺ = 262) par spectrométrie de masse par « fast atomic bombardment ».

L'analyse élémentaire du carbone, proton et azote correspond à la masse molaire 261 et à la formule C₁₃H₁₅N₃O₃.

### Activité biologique

On a comparé l'activité biologique du dérivé de formule I avec le dipropylènetriamine NONOate (A), la mélatonine (B) et un mélange dipropylènetriamine NONOate et mélatonine (1/2) (C) sur la préparation cellulaire de la prolifération cellulaire de la lignée P 815.

Les cellules sont incubées avec les produits dans le milieu RPMI avec rouge de phénol durant 7 heures. Le thymidine tritiée est ajoutée après 3 heures d'incubation.

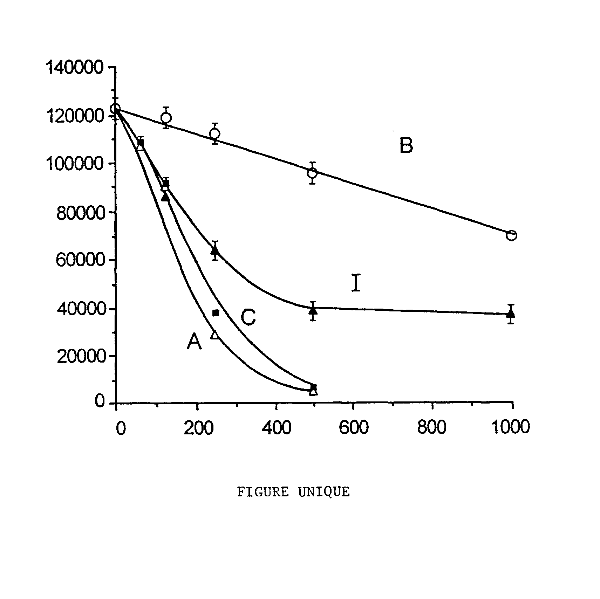

Les résultats sont indiqués sur la courbe de la figure unique ci-jointe où est portée en abscisse la concentration de produit (µM) et en ordonnée la radioactivité associée à l'ADN (cpm).

L'IC₅₀ pour le composé de formule I se produit pour une concentration de 280 µM, l'IC₅₀ pour le dipropylènetriamine NONOate (A) est de 150 µM.

On constate donc que le dérivé de mélatonine selon l'invention révèle une activité comparable à celle du dipropylènetriamine NONOate.

## Revendications

1. Dérivé de mélatonine de formule : 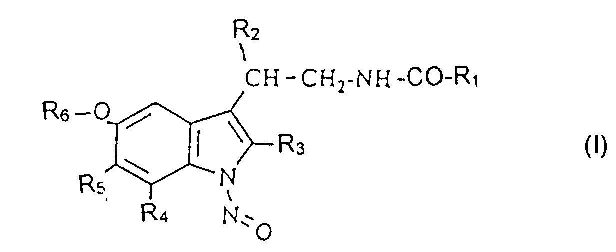 dans laquelle :
R₁ représente H, un groupe alkyle en C₁ à C₄ ou un groupe alcoxy en C₁ à C₄.
R₂ représente H ou un groupe alkyle en C₁ à C₄
R₃ représente H, méthyle ou un atome d'halogène,
R₄, R₅ représentent individuellement H ou un atome d'halogène,
R₆ représente H ou un groupe alkyle en C₁-C₄
ou un sel pharmaceutiquement acceptable de celui-ci.

2. Dérivé de mélatonine selon la revendication 1, **caractérisé en ce que** R₂= H ; R₆ = CH₃ ; R₁ = CH₃.

3. Dérivé de mélatonine selon la revendication 2 de formule

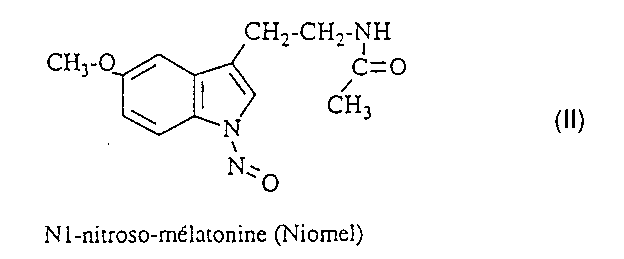

4. Dérivé de mélatonine selon l'une des revendications 1 à 3, pour son application en tant que substance thérapeutiquement active.

5. Dérivé de mélatonine selon l'une des revendications 1 à 3, pour son application en tant que substance thérapeutiquement active dans le traitement des maladies cardio-vasculaires, les ischémie-reperfusions, les maladies parasitaires, les maladies inflammatoires, les maladies neurodégénératives notamment la maladie d'Alzheimer, les myopathies, les anémies falciformes.

6. Composition pharmaceutique **caractérisée en ce qu'**elle comprend un dérivé de mélatonine de formule I selon la revendication 1 et un excipient pharmaceutique actif.

7. Procédé de préparation d'un dérivé de mélatonine selon la revendication 1, **caractérisé en ce que** l'on met en contact en solution organique protique un précurseur de formule : 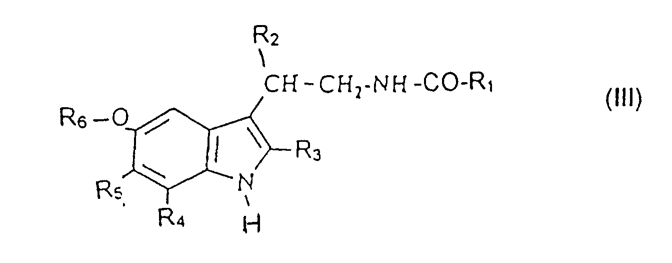 dans laquelle R₁ à R₆ ont les mêmes significations que dans la revendication 1,
avec une solution aqueuse de nitrite de sodium puis on alcalinise de façon à obtenir un dérivé selon l'une des revendications 1 à 3.

## Patentansprüche

1. Melatonin-Derivat der Formel: 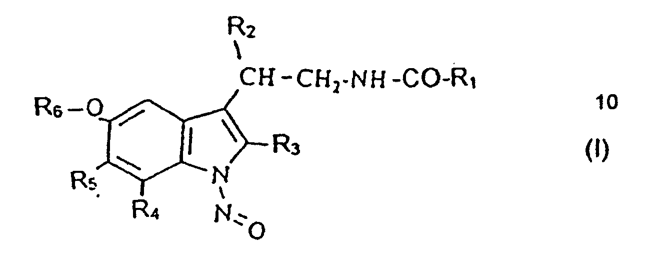 in der:
R₁ für H, eine C₁- bis C₄-Alkylgruppe oder eine C₁- bis C₄-Alkoxygruppe steht,
R₂ für H oder eine C₁- bis C₄-Alkylgruppe steht,
R₃ für H, Methyl oder ein Halogenatom steht,
R₄, R₅ einzeln H oder ein Halogenatom darstellen,
R₆ für H oder eine C₁- bis C₄-Alkylgruppe steht,
oder ein pharmazeutisch annehmbares Salz desselben.

2. Melatonin-Derivat nach Anspruch 1, **dadurch gekennzeichnet, dass** R₂ = H; R₆ = CH₃; R₁ = CH₃.

3. Melatonin-Derivat nach Anspruch 2 der Formel:

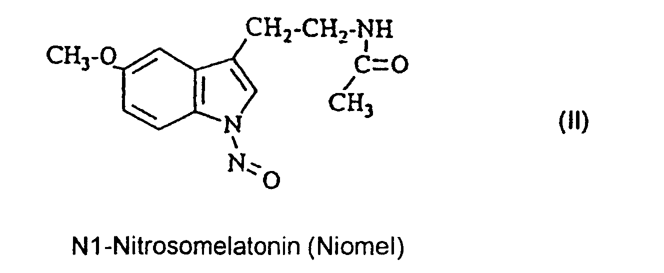

4. Melatonin-Derivat nach einem der Ansprüche 1 bis 3 für seine Anwendung als therapeutisch wirksame Substanz.

5. Melatonin-Derivat nach einem der Ansprüche 1 bis 3 für seine Anwendung als therapeutisch wirksame Substanz bei der Behandlung von kardiovaskulären Krankheiten, Ischämiereperfusionen, parasitären Krankheiten, entzündlichen Krankheiten, neurodegenerativen Krankheiten, insbesondere der Alzheimer-Krankheit, Myopathien, Sichelanämien.

6. Pharmazeutische Zusammensetzung, **dadurch gekennzeichnet, dass** sie ein Melatonin-Derivat der Formel I nach Anspruch 1 und einen pharmazeutischen aktiven Träger umfasst.

7. Verfahren zur Herstellung eines Melatonin-Derivats nach Anspruch 1, **dadurch gekennzeichnet, dass** man eine Vorstufe der Formel: 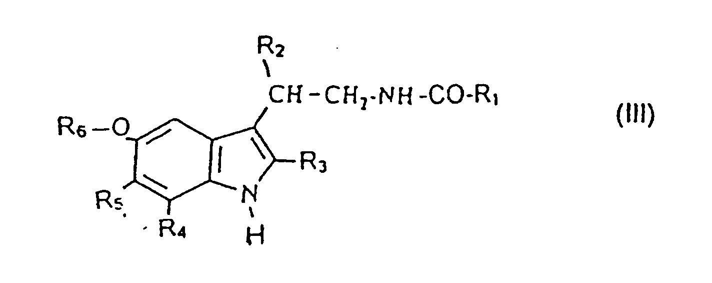 in der R₁ bis R₆ die gleichen Bedeutungen wie in Anspruch 1 aufweisen, in einer protischen organischen Lösung mit einer wässrigen NatriumnitritLösung in Kontakt bringt, dann auf solche Weise alkalisiert, dass man ein Derivat nach einem der Ansprüche 1 bis 3 erhält.

## Claims

1. Melatonin derivative of formula: 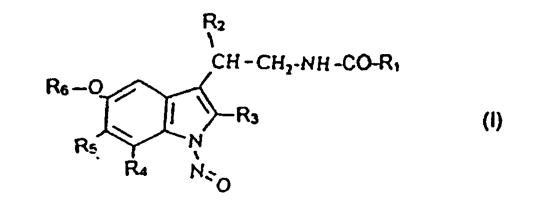 in which:
R₁ represents H, a C₁ to C₄ alkyl group or a C₁ to C₄ alkoxy group,
R₂ represents H or a C₁ to C₄ alkyl group,
R₃ represents H, methyl or a halogen atom,
R₄ and R₅ individually represent H or a halogen atom,
R₆ represents H or a C₁ to C₄ alkyl group,
or a pharmaceutically acceptable salt of the latter.

2. Melatonin derivative according to Claim 1, **characterized in that** R₂ = H, R₆ = CH₃ and R₁ = CH₃.

3. Melatonin derivative according to Claim 2 of formula:

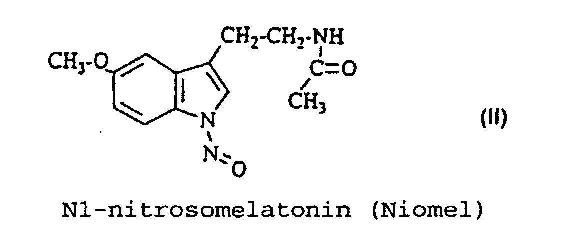

4. Melatonin derivative according to one of Claims 1 to 3, for its application as therapeutically active substance.

5. Melatonin derivative according to one of Claims 1 to 3, for its application as therapeutically active substance in the treatment of cardiovascular diseases, ischaemia reperfusion injuries, parasitic diseases, inflammatory diseases, neurodegenerative diseases, in particular Alzheimer's disease, myopathies or falciform anaemias.

6. Pharmaceutical composition, **characterized in that** it comprises a melatonin derivative of formula I according to Claim 1 and a pharmacologically acceptable excipient.

7. Process for the preparation of a melatonin derivative according to Claim 1, **characterized in that** a precursor of formula: 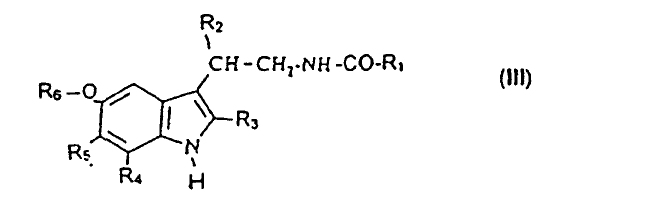 in which R₁ to R₆ have the same meanings as in Claim 1,
is brought into contact, in protic organic solution, with an aqueous sodium nitrite solution and then the mixture is basified, so as to obtain a derivative according to one of Claims 1 to 3.
